Europäisches Patentamt

European Patent Office

Office européen des brevets

(11) Numéro de publication : **0 306 409 B1**

## (12) FASCICULE DE BREVET EUROPEEN

(45) Date de publication du fascicule du brevet :
**15.01.92 Bulletin 92/03**

(21) Numéro de dépôt : **88402200.5**

(22) Date de dépôt : **01.09.88**

(51) Int. Cl.⁵ : **G01B 11/24,** A61F 9/00,
A61B 3/10

(54) **Système optique pour déterminer la variation de courbure d'un objet sur une zone de petites dimensions.**

(30) Priorité : **04.09.87 FR 8712302**

(43) Date de publication de la demande :
**08.03.89 Bulletin 89/10**

(45) Mention de la délivrance du brevet :
**15.01.92 Bulletin 92/03**

(84) Etats contractants désignés :
**AT BE CH DE ES GB GR IT LI LU NL SE**

(56) Documents cités :
**WO-A-86/02249**
**GB-A- 1 209 451**
**US-A- 4 209 252**
**PATENT ABSTRACTS OF JAPAN, vol. 8, no. 42**
**(P-256) [1479], 23 février 1984, page 28 P 256; &**
**JP-A-58 193 408 (NTN TOYO BEARING K.K.)**
**11.11.1983**

(73) Titulaire : **Azema, Alain**
**28 avenue des Arènes**
**F-06000 Nice (FR)**

Titulaire : **Arneodo, Jacques**
**55 Promenade des Anglais**
**F-06000 Nice (FR)**
Titulaire : **Botineau, Jean**
**Ile Verte 11 Place des Genèvriers**
**F-06560 Valbonne (FR)**
Titulaire : **Crozafon, Philippe**
**55, Promenade des Anglais**
**F-06000 Nice (FR)**
Titulaire : **Moulin, Gérard**
**4 Place Méjane Sophia Antipolis**
**F-06560 Valbonne (FR)**

(72) Inventeur : **Azema, Alain**
**28 avenue des Arènes**
**F-06000 Nice (FR)**
Inventeur : **Moulin, Gérard**
**167 avenue Maréchal Lyautey**
**F-06000 Nice (FR)**
Inventeur : **Botineau, Jean**
**5 avenue Chateaubriand**
**F-06000 Nice (FR)**

(74) Mandataire : **Bonnetat, Christian et al**
**CABINET BONNETAT 23, Rue de Léningrad**
**F-75008 Paris (FR)**

## Description

La présente invention concerne un système optique pour déterminer la courbure ou la modification apportée à la courbure d'un objet.

L'invention s'applique plus particulièrement, mais non exclusivement, à la détermination de la variation de la courbure de la cornée oculaire, dans sa zone centrale, au cours d'un traitement chirurgical de celle-ci, notamment par photoablation, à l'aide, par exemple, de l'appareil décrit dans le document EP-A-0191688, et spécialement adapté au traitement de zones n'excédant pas quelques millimètres de diamètre.

Un tel appareil chirurgical est destiné à modifier la courbure de la cornée oculaire par photodécomposition de la matière cornéenne. On sait, en effet, que certaines affections, telles que la myopie, l'hypermétropie et l'astigmatisme, peuvent être traitées par modification de la courbure de la cornée.

Lors d'un tel traitement chirurgical, il est essentiel de pouvoir déterminer, de façon précise, fiable et rapide, la variation, au cours du traitement, de la courbure de la zone centrale de la cornée afin, à chaque étape élémentaire du traitement, d'une part, de mesurer l'ablation effective de matière cornéenne lors de ladite étape, et, d'autre part, de moduler, en fonction de ladite ablation, l'amplitude de l'étape suivante, étant bien entendu qu'il est crucial de ne pas dépasser, en fin de traitement, une modification de courbure prédéterminée.

Par le document WO-A-86/02249, on connaît déjà un système optique pour déterminer la courbure ou la variation de courbure d'un objet dans une zone de petit diamètre, ledit système optique comprenant :
— une source lumineuse ;
— un élément semi-réfléchissant disposé sur le trajet d'un premier faisceau lumineux provenant de ladite source lumineuse et transmettant un second faisceau lumineux ;
— une seconde optique de focalisation recevant ledit second faisceau lumineux et focalisant celui-ci en direction dudit objet, qui réfléchit ledit second faisceau lumineux en direction de ladite optique de focalisation de façon à former un troisième faisceau adressé audit élément semi-réfléchissant ;
— une troisième optique de focalisation recevant ledit troisième faisceau provenant dudit élément semi-réfléchissant et focalisant ledit troisième faisceau ;
— des moyens de détection recevant ledit troisième faisceau focalisé, qui forme une image dudit objet sur lesdits moyens de détection ; et
— des moyens d'affichage reliés auxdits moyens de détection.

Dans ce dispositif connu, ladite seconde optique est constituée par un élément holographique permettant la détermination de la courbure d'une cornée réelle par comparaison avec une forme théorique.

La présente invention concerne un système optique permettant de déterminer, de façon précise, simple, fiable et rapide, sans recours à un élément holographique, la modification de courbure de la cornée au cours d'un traitement chirurgical, et, plus généralement, celle d'un objet dont la courbure doit subir des modifications.

A cet effet, le système optique du type décrit ci-dessus est caractérisé en ce que :
— il comporte une première optique destinée à transformer le faisceau émis par ladite source en un faisceau parallèle constituant ledit premier faisceau lumineux ;
— lesdits second et troisième faisceaux lumineux sont des faisceaux parallèles ;
— ladite seconde optique de focalisation est une lentille convergente et est susceptible de se déplacer parallèlement à la direction de propagation dudit second faisceau lumineux, de façon que son foyer image puisse être amené à coïncider au moins sensiblement avec le centre de courbure ou la surface dudit objet ; et
— lesdits moyens d'affichage affichent ladite image, ladite seconde optique étant déplacée entre deux positions pour lesquelles les images correspondantes sont nettes et ladite courbure ou variation de courbure étant déduite du déplacement de ladite seconde optique entre lesdites deux positions.

On voit que, dans le système optique de l'invention, les dispositions de la source lumineuse et des moyens de détection sont interchangeables.

Selon un premier mode de fonctionnement (kératométrie), ladite deuxième optique de focalisation est placée selon deux positions telles que son foyer image coïncide successivement avec le centre de courbure de l'objet et avec la surface de l'objet, les réglages étant effectués par la meilleure mise au point de l'image. Le déplacement de la deuxième optique entre ces deux positions fournit la valeur du rayon de courbure (et éventuellement de l'astigmatisme de la surface mesurée).

Selon un deuxième mode de fonctionnement (kératométrie différentielle), si, initialement, ladite seconde optique de focalisation est placée de façon telle que son foyer image coïncide avec le centre de courbure de l'objet (meilleure mise au point de l'image), et si ce dernier subit une modification de courbure, la seconde optique peut alors être déplacée jusqu'à ce que le foyer image de celle-ci coïncide avec le nouveau centre de courbure de l'objet (image nette), et la mesure de ce déplacement permettra de déterminer la modification de courbure correspondante de l'objet.

Selon une autre caractéristique de l'invention, lesdits moyens de détection sont constitués d'ensembles de photodiodes.

Selon encore d'autres caractéristiques de l'invention, ladite troisième optique de focalisation est associée à une quatrième optique de focalisation disposée entre ladite troisième optique de focalisation et ledit ensemble de photodiodes, permettant ainsi la formation d'une image de dimension adaptée à celle de l'ensemble de photodiodes et ladite troisième optique de focalisation est une lentille convergente.

De plus, ladite source lumineuse peut être constituée par une diode laser ou une diode électroluminescente, éventuellement associée à une fibre optique.

Par ailleurs, des moyens d'affichage de l'image formée par le système peuvent être associés auxdits moyens de détection.

Les figures du dessin annexé feront bien comprendre comment l'invention peut être réalisée.

La figure 1 est une vue schématique du système optique selon l'invention.

La figure 2 est une vue agrandie de la zone A de la figure 1 illustrant le fonctionnement du système de l'invention.

Le système optique de l'invention, pour déterminer la variation de courbure d'un objet, notamment une cornée oculaire au cours d'un traitement chirurgical de correction de courbure de celle-ci, par exemple par photodécomposition, comprend une source 1 susceptible d'émettre un faisceau lumineux 2a. Essentiellement pour des raisons d'ordre énergétique et, également, pour obtenir facilement une source ponctuelle, il est avantageux d'utiliser comme source lumineuse une diode laser ou une diode électroluminescente, éventuellement couplée à une fibre optique.

Le système optique comprend ensuite une première optique 3, constituée, par exemple, par une lentille collimatrice, destinée à former un faisceau lumineux parallèle 2b à partir du faisceau 2a émis par la source 1.

Un miroir semi-réfléchissant 4 est disposé sur le trajet du faisceau parallèle 2b. Le miroir 4 est incliné, dans cet exemple de réalisation, à 45° par rapport à l'axe optique 5 du faisceau lumineux 2b reçu par le miroir 4, de sorte que l'axe optique 6 du faisceau 2c réfléchi par le miroir 4 est perpendiculaire à l'axe optique 5 du faisceau 2b reçu par le miroir 4.

Une deuxième optique de focalisation 7, constituée, par exemple, par une lentille convergente, reçoit le faisceau parallèle 2c réfléchi par le miroir 4 et est susceptible de se déplacer, parallèlement à la direction de propagation du faisceau lumineux 2c réfléchi par le miroir 4 (flèche double F), de façon que son foyer image 8 coïncide au moins sensiblement avec le centre de courbure C de l'objet 9. Ce dernier réfléchit le faisceau lumineux 2c en direction de la lentille 7 de façon à former un faisceau 2d traversant le miroir 4.

Une troisième optique de focalisation constituée, dans l'exemple représenté, par une lentille convergente 10 et un objectif de microscope 14, reçoit le faisceau 2d ayant traversé le miroir 4, et des moyens de détection 11 sont prévus au-delà de la troisième optique 10, dans le sens de propagation du faisceau réfléchi 2d, pour détecter l'image 12 formée par le système. Dans l'exemple représenté, les moyens de détection 11 sont constitués par une barrette de photodiodes. Par ailleurs, des moyens d'affichage 15 de l'image formée par le système peuvent être associés aux moyens de détection 11. On notera que les dispositions des éléments 1-2a, 3-2b-5 et 2d-10-14-12-15 peuvent être échangées.

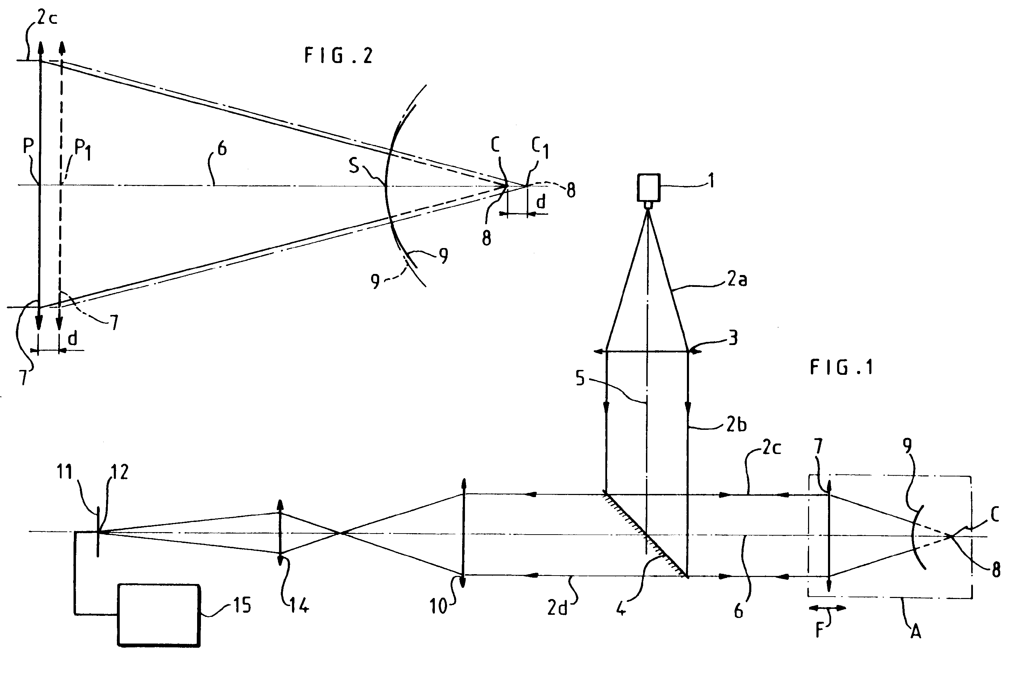

Le fonctionnement du système optique selon l'invention sera décrit ci-après, en se référant plus particulièrement à la figure 2.

Dans un premier mode de fonctionnement (kératométrie), la lentille 7 se trouve en une certaine position P le long de l'axe optique 6 pour laquelle le foyer image 8 de la lentille 7 coïncide avec le centre de courbure C de l'objet 9. Dans cette position de la lentille 7, l'objet 9 réfléchit le faisceau 2c en un faisceau parallèle 2d identique au précédent au sens près de propagation de la lumière, de sorte que l'on obtient la meilleure mise au point de l'image 12 formée par le système sur la barrette 13 de photodiodes. Cette meilleure mise au point se traduit par l'éclairement d'un nombre minimum de photodiodes, indiqué par les moyens d'affichage 15. Pour une autre position Ps de la lentille 7 (non représentée) le long de l'axe optique 6, le foyer image 8 de la lentille 7 coïncide avec le sommet S de l'objet 9, ce qui correspond de nouveau à une image nette sur le détecteur 11. La distance entre les points P et P' donne le rayon de courbure de l'objet C au point S. Si l'objet C n'est pas sphérique en S, on pourra mesurer son astigmatisme par l'écart entre les deux points P' et P'' correspondant à la meilleure mise au point sur les centres de courbure principaux.

Dans un deuxième mode de fonctionnement (kératométrie différentielle), on commence comme ci-dessus le réglage de la lentille 7 au point P correspondant au centre de courbure de l'objet C au point S. Quand l'objet 9 subit une modification de courbure (sur la figure 2, on a supposé que le sommet S était conservé), l'image formée par le système est modifiée, ce qui peut se lire sur les moyens d'affichage. L'opérateur déplacera alors la lentille 7 de façon à tendre vers l'éclairement d'un minimum de photodiodes indiqué ci-dessus. La position P1 de la lentille 7 correspondant à cet éclairement minimum sera telle que le foyer image 8 de celle-ci coïncide avec le nouveau centre de courbure C1 de l'objet 9. En mesurant le déplacement $d$ correspondant de la lentille 7, on détermine la variation de la courbure de l'objet 9.

Le déplacement de la lentille 7 peut être effectué manuellement, ou à l'aide de moyens moteurs (non représentés) éventuellement couplés aux moyens de détection de l'image et susceptibles de commander le déplacement de la lentille, pour une meilleure mise au point de l'image, en fonction de l'image détectée.

Le système optique de l'invention, connu sous le nom de "kératomètre", peut être intégré à un appareil chirurgical de modification de la courbure de la cornée oculaire. Les informations fournies par le kératomètre permettent, après traitement sur un calculateur, notamment de moduler l'amplitude de l'étape élémentaire suivante de traitement en fonction des résultats ainsi mesurés de l'étape précédente.

**Revendications**

1. Système optique pour déterminer la courbure ou la variation de courbure d'un objet (9) dans une zone de petit diamètre, ledit système optique comprenant :
— une source lumineuse (1) ;
— un élément semi-réfléchissant (4) disposé sur le trajet d'un premier faisceau lumineux (2b) provenant de ladite source lumineuse (1) et transmettant un second faisceau lumineux (2c) ;
— une seconde optique de focalisation (7) recevant ledit second faisceau lumineux (2c) et focalisant celui-ci en direction dudit objet (9), qui réfléchit ledit second faisceau lumineux (2c) en direction de ladite optique de focalisation de façon à former un troisième faisceau (2d) adressé audit élément semi-réfléchissant (4) ;
— une troisième optique de focalisation (10) recevant ledit troisième faisceau (2d) provenant dudit élément semi-réfléchissant (4) et focalisant ledit troisième faisceau (2d) ;
— des moyens de détection (11) recevant ledit troisième faisceau (2d) focalisé, qui forme une image (12) dudit objet (9) sur lesdits moyens de détection (11) ; et
— des moyens d'affichage (15) reliés auxdits moyens de détection (11) ; caractérisé en ce que:
— il comporte une première optique (3) destinée à transformer le faisceau (2a) émis par ladite source en un faisceau parallèle constituant ledit premier faisceau lumineux (2b) ;
— lesdits second et troisième faisceau lumineux (2c) et (2d) sont des faisceaux parallèles ;
— ladite seconde optique de focalisation (7) est une lentille convergente et est susceptible de se déplacer parallèlement à la direction de propagation dudit second faisceau lumineux (2c), de façon que son foyer image (8) puisse être amené à coïncider au moins sensiblement avec le centre de courbure (C) ou la surface (S) dudit objet (9) ; et
— lesdits moyens d'affichage (15) affichent ladite image (12), ladite seconde optique (7) étant déplacée entre deux positions pour lesquelles les images (12) correspondantes sont nettes et ladite courbure ou variation de courbure étant déduite du déplacement de ladite seconde optique (7) entre lesdites deux positions.

2. Système optique selon la revendication 1, caractérisé en ce que lesdits moyens de détection (11) sont constitués d'ensembles de photodiodes.

3. Système optique selon la revendication 2, caractérisé en ce que ladite troisième optique de focalisation (10) est associée à une quatrième optique de focalisation (14) disposée entre ladite troisième optique de focalisation (10) et ledit ensemble de photodiodes (11), permettant ainsi la formation d'une image de dimension adaptée à celle de l'ensemble de photodiodes (11).

4. Système optique selon l'une quelconque des revendications 1 à 3, caractérisé en ce que ladite troisième optique (10) de focalisation est une lentille convergente.

5. Système optique selon l'une quelconque des revendications 1 à 4, caractérisé en ce que ladite première optique (3) est une lentille collimatrice.

6. Système optique selon l'une quelconque des revendications 1 à 5, caractérisé en ce que ladite source lumineuse (1) est constituée par une diode laser ou une diode électroluminescente, éventuellement associée à une fibre optique.

7. Système optique selon l'une quelconque des revendications 1 à 6, caractérisé en ce que les moyens d'affichage (15) de l'image formée par le système sont associés auxdits moyens de détection (11).

**Claims**

1. Optical system for determining the curvature or the variation of curvature of an object (9) in a zone of small diameter, said optical system comprising :
— a light source (1) ;
— a semi-reflecting element (4) disposed in the path of a first light beam (2b) coming from said light source (1) and transmitting a second light beam (2c) ;
— second focussing optics (7) receiving said second light beam (2c) and focussing it in direction of said object (9), which reflects said second light beam (2c) in direction of said focussing optics so as to form a third beam (2d) addressed to said semi-reflecting element (4) ;
— third focussing optics (10) receiving said third beam (2d) coming from said semi-reflecting element (4) and focussing said third beam (2d) ;
— detection means (11) receiving said third focussed beam (2d), which forms an image (12) of said object (9) on said detection means (11) ; and
— displaying means (15) connected to said detection means (11) ; characterized in that :

— it comprises first optics (3) adapted to transform the beam (2a) emitted by said light source into a parallel beam constituting said first light beam (2b) ;

— said second and third light beams (2c) and (2d) are parallel beams ;

— said second focussing optics (7) is a focussing lens and is capable to move in a direction parallel to the direction of propagation of said second light beam (2c), so that its image focus (8) may be brought in coincidence at least substantially with the centre of curvature (C) or the surface (S) of said object (9) ; and

— said displaying means (15) display said image (12), said second optics (7) being moved between two positions for which the corresponding images (12) are sharp and said curvature or variation of curvature being deduced from the displacement of said second optics (7) between said two positions.

2. Optical system according to claim 1, characterized in that said detection means (11) are constituted by assemblies of photodiodes.

3. Optical system according to claim 2, characterized in that said third focussing optics (10) is associated with a fourth focussing optics (14) disposed between said third focussing optics (10) and said assembly of photodiodes (11), allowing thus the formation of an image having a dimension adapted to that of the assembly of photodiodes.

4. Optical system according to anyone of claims 1 to 3, characterized in that said third focussing optics (10) is a focussing lens.

5. Optical system according to anyone of claims 1 to 4, characterized in that said first focussing optics (3) is a collimator lens.

6. Optical system according to anyone of claims 1 to 5, characterized in that said light source (1) is constituted by a laser diode or an electroluminescent diode, possibly associated with an optical fiber.

7. Optical system according to anyone of claims 1 to 6, characterized in that means (15) for displaying the image formed by the system are associated with said detection means (11).

**Patentansprüche**

1. Optisches System zur Bestimmung der Krümmung oder der Änderung der Krümmung eines Objekts (9) in einer Zone kleinen Durchmessers mit :

— einer Lichtquelle (1) ;

— einem halbreflektierenden Element (4), das in dem Strahlenweg eines von der Lichtquelle (1) ausgehenden Strahlenbündels (2b) angeordnet ist und ein zweites Strahlenbündel (2c) weiterleitet ;

— einer zweiten Fokussierungsoptik (7), welche das zweite Strahlenbündel (2c) empfängt und dieses in Richtung auf das Objekt (9) fokussiert, welches das zweite Strahlenbündel (2c) in Richtung zur Fokussierungsoptik in der Weise reflektiert, daß ein drittes auf das halbreflektierende Element (4) gerichtetes Strahlenbündel (2d) gebildet wird ;

— einer dritten Fokussierungsoptik (10), die das von dem halbreflektierenden Element (4) kommende dritte Strahlenbündel (2d) empfängt und fokussiert ;

— Erfassungsmittel (11), welche das dritte fokussierte Strahlenbündel (2d) empfangen, das auf den Erfassungsmitteln (11) ein Bild (12) des Objekts (9) erzeugt ; und

— mit den Erfassungsmitteln (11) verbundene Anzeigemittel (15) ; dadurch gekennzeichnet,

— daß es eine erste Optik (3) aufweist, die dazu bestimmt ist, das von der Lichtquelle ausgesandte Bündel (2a) in ein paralleles Bündel, das Strahlenbündel (2b), zu transformieren ;

— daß das zweite und dritte Strahlenbündel (2c) und (2d) parallele Strahlenbündel sind ;

— daß die zweite Fokussierungsoptik (7) eine Sammellinse und imstande ist, sich parallel zur Ausbreitungsrichtung des zweiten Strahlenbündels (2c) in der Weise zu bewegen, daß sein bildseitiger Brennpunkt (8) zumindest annähernd mit dem Krümmungsmittelpunkt (C) oder der Oberfläche (S) des Objekts (9) zur Koinzidenz gebracht werden kann ; und

— daß die Anzeigemittel (15) das Bild (12) anzeigen, wobei die zweite Optik (7) zwischen zwei Positionen bewegt wird, in denen die entsprechenden Bilder (12) scharf sind, und die Krümmung oder Änderung der Krümmung von dem Verstellweg der zweiten Optik (7) zwischen den beiden genannten Positionen abgeleitet wird.

2. Optisches System nach Anspruch 1, dadurch gekennzeichnet, daß die Erfassungsmittel (11) als Fotodiodenanordnung ausgebildet sind.

3. System nach Anspruch 2, dadurch gekennzeichnet, daß die dritte Fokussierungsoptik (10) mit einer vierten Fokussierungsoptik (14) verbunden ist, die zwischen der dritten Fokussierungsoptik (10) und der Diodenanordnung (11) vorgesehen ist und so die Erzeugung eines Bildes erlaubt, dessen Dimension derjenigen der Fotodiodenanordnung (11) angepaßt ist.

4. Optisches System nach einem der vorhergehenden Ansprüche 1 bis 3, dadurch gekennzeichnet, daß die dritte Fokussierungsoptik (10) eine Sammellinse ist.

5. Optisches System nach einem der vorhergehenden Ansprüche 1 bis 4, dadurch gekennzeichnet, daß die erste Optik (3) eine Kollimatorlinse ist.

6. Optisches System nach einem der vorhergehenden Ansprüche 1 bis 5, dadurch gekennzeichnet, daß die Lichtquelle (1) als Laserdiode oder Elektrolumineszenzdiode ausgebildet und eventuell mit einer optischen Faser verbunden ist.

7. Optisches System nach einem der vorhergehenden Ansprüche 1 bis 6, dadurch gekennzeichnet, daß die Anzeigemittel (15) des von dem System erzeugten Bildes mit den Erfassungsmitteln (11) verbunden sind.

FIG.2

FIG.1

EP 0 306 409 B1